# EUROPÄISCHE PATENTSCHRIFT

(11) **EP 3 911 216 B1**
(45) Veröffentlichungstag und Bekanntmachung des Hinweises auf die Patenterteilung: **01.05.2024**
(21) Anmeldenummer: 20703136.0
(22) Anmeldetag: 16.01.2020
(51) Int. Cl.: A61B 1/005, A61B 17/00

(54) **ENDOSKOPISCHE SONDE MIT AKTUATOR**
ENDOSCOPIC PROBE WITH ACTUATOR
SONDE ENDOSCOPIQUE AVEC ACTIONNEUR

(30) Priorität: 16.01.2019 DE 102019101089
(43) Veröffentlichungstag der Anmeldung: 24.11.2021
(73) Patentinhaber: viZaar Industrial imaging AG, 72461 Albstadt (DE)
(72) Erfinder: ZAAR, Björn Simon, 72461 Albstadt (DE)
(74) Vertreter: Geitz Patentanwälte PartG mbB
(86) Internationale Anmeldenummer: PCT/DE2020/100029
(87) Internationale Veröffentlichungsnummer: WO 2020/147894

(56) Entgegenhaltungen:
- JP-A- H01 264 795
- US-A- 4 930 494
- US-A- 5 531 664

## Beschreibung

Die Erfindung geht aus von einer endoskopischen Sonde.

Endoskopische Sonden werden im technischen oder im medizinischen Bereich eingesetzt, um Strukturen in schwer zugänglichen Hohlräumen zu untersuchen. Hierzu werden die mit einem länglichen, flexiblen Schaft ausgestatteten Sonden in den Hohlraum eingeführt. Der Schaft weist an seinem vorderen Ende, welches auch als distales Ende bezeichnet wird, einen Schaftkopf auf. Der Schaftkopf kann insbesondere mit einer Kamera ausgestattet sein. Darüber hinaus können eine Lichtquelle und/ oder ein Werkzeug an dem Schaftkopf angeordnet sein. Der Schaft der Sonde weist in der Regel einen Kanal auf, in welchem Versorgungsleitungen für die Kamera, die Lichtquelle und gegebenenfalls für Werkzeuge geführt werden können. Eine endoskopische Sonde unterscheidet sich von einem starren Endoskopschaft dadurch, dass sie flexibel ist und sich daher auch in Hohlräume einführen läßt, deren Zugang abgewinkelt ist. Als Bildgeber dienen häufig Bildsensoren der CCD oder CMOS Kameratechnik, welche direkt am Schaftkopf angeordnet sind. Eine traditionelle Bildübertagung via gerichtetes Bildbündel entfällt.

Damit der Hohlraum besser untersucht werden kann, sind endoskopische Sonden mit einem Schaftkopf ausgestattet, der mittels mindestens eines Aktuators gegenüber dem Schaft bewegt werden kann. Dies erleichtert es, eine an dem Schaftkopf angeordnete Kamera, eine Lichtquelle und/ oder ein Werkzeug in dem Hohlraum auszurichten. Aus dem Stand der Technik sind hierzu mit Druckfluiden betätigte Aktuatoren (EP 0 304 380 B1) und mit Formgedächtnislegierungen ausgestattete Aktuatoren (EP 2 042 076 B2, DE 38 74 122 T2) bekannt. Die mit Druckfluiden betätigten Aktuatoren weisen den Nachteil auf, dass ein Druckfluid in der Sonde zur Verfügung gestellt werden muss, was entsprechende Zuleitungen notwendig macht und in der Sonde Platz beansprucht. Die mit Formgedächtnislegierungen ausgestatteten Aktuatoren weisen den Nachteil auf, dass die Formänderung der Formgedächtnislegierungen eine Erwärmung auf eine gewisse Temperatur erfordert, welche jedoch häufig am Einsatzort der Sonde unerwünscht ist. So müssen die Formgedächtnislegierungen zunächst mittels einer Heizeinrichtung erwärmt werden oder mittels eines elektrischen Leiters mit Strom versorgt werden, so dass der durch die Formgedächtnislegierung fließende Strom zu einer Erwärmung der Legierung fürht. Je nach Einsatzgebiet sind zusätzliche Kühlvorrichtungen notwendig, welche für einen aufwändigen Aufbau sorgen und Platz beanspruchen. Die Druckfluide und die Kühleinrichtungen sorgen zum einen für einen größeren Querschnitt der Sonde und begrenzen andererseits die Sondenlänge. Dokument US 5 531 664 A offenbart eine endoskopische Sonde gemäß des Oberbegriffs des Anspruchs 1.

Der Erfindung liegt die Aufgabe zugrunde, einen Aktuator für eine endoskopische Sonde und eine endoskopische Sonde mit Aktuator zur Verfügung zu stellen, die keine Druckfluide und Kühlreinrichtungen benötigen, bei denen die Sonde nicht auf eine Temperatur oberhalb einer vorgegebenen Schwellentemperatur erwärmt wird, die zuverlässig über eine Vielzahl von Zyklen eine Einstellung des Schaftkopfes gegenüber dem Schaft ermöglichen, sowohl kleine als auch große Schaftdurchmesser erlauben und die die Sondenlänge nicht begrenzen.

Diese Aufgabe wird durch eine endoskopische Sonde mit den Merkmalen des Anspruchs gelöst. Jeder der mindestens zwei Aktuatoren der endoskopischen Sonde mit den Merkmalen des Anspruchs 1 zeichnet sich dadurch aus, dass er mit einem länglichen Formspeicher-Draht aus einer Formgedächtnislegierung, einem elektrischen Leiter, einer den FormspeicherDraht umgebenden Draht-Isolierung und einer die Draht-Isolierung umgebenden flexiblen und in Längsrichtung des Formspeicher-Drahtes druckfesten Hülle ausgestattet ist. Dabei ist der elektrische Leiter elektrisch leitend mit dem Formspeicher-Draht verbunden und versorgt den Formspeicher-Draht mit Strom. Der Formspeicher-Draht ist derart ausgebildet, dass er die elektrische Energie des durch ihn fließenden Stroms in Wärmeenergie umwandelt und sich unter Einfluss dieser Wärme die Länge des Formspeicher-Drahtes ändert. Dabei geht die in Längsrichtung gemessene Länge des Formspeicher-Drahtes von einer ersten Länge in eine zweite Länge über. In bevorzugter Weise erfolgt eine Längenkontraktion. Hierzu wird bevorzugt eine Formgedächtnislegierung gewählt, die bei einem aus ihr hergestellten Formspeicher-Draht die Eigenschaft aufweist, dass sich dieser bei Überschreiten einer vorgegebenen Temperatur zusammenzieht. Die den Formspeicher-Draht zumindest abschnittweise umgebende Draht-Isolierung besteht aus einem elektrisch isolierenden Material. Sie sorgt dafür, dass der Strom von dem Formspeicher-Draht nicht unerwünscht und unkontrolliert in die Umgebung des Formspeicher-Drahtes abfließt. Die Draht-Isolierung isoliert den Formspeicher-Draht auch gegen die Hülle. Dabei wird verhindert, dass ein elektrischer Strom an die Hülle abgegeben wird. Die Hülle bildet eine Führung für den Formspeicherdraht und ein Gegenlager zur Abstützung der zu übertragenden, durch die Formänderung des Formspeicherdrahtes wirkenden Zugkräfte. Im Unterschied zu dem Formspeicher-Draht bleibt die Länge der Hülle in Längsrichtung im wesentlichen konstant. Durch eine Längenänderung des Formspeicher-Drahtes relativ zu der den Formspeicher-Draht umgebenden Hülle können mittels des Aktuators mechanische Bewegungen sowie Zug- oder Druckkräfte übertragen werden. Erfindungsgemäß sind die mindestens zwei Aktuatoren in dem Schaft derart angeordnet, dass die Formspeicher-Drähte der Aktuatoren in axialer Richtung des Schaftes versetzt zueinander und frei von einer Überlappung in axialer Richtung sind.

Mindestens ein Ende der Hülle sowie ein Ende des Formspeicher-Drahtes können mechanisch festgelegt sein, so dass sich ihre Position relativ zueinander nicht verändert. Hierzu kann ein Gegenhalter vorgesehen sein. Der Gegenhalter kann eine erste Gegenhalter-Komponente zur Festlegung des FormspeicherDrahtes und eine zweite Gegenhalter-Komponente zur Festlegung der Hülle aufweisen. Bei einer Längenänderung des Formspeicher-Drahtes verschiebt sich nun das dem Gegenhalter abgewandte Ende des Formspeicher-Drahtes relativ zu dem dem Gegenhalter abgewandten Ende der Hülle. Es wird beispielsweise weiter in die Hülle hineingezogen oder aus der Hülle herausgeschoben. Der dabei zurückgelegte Weg kann auf einen Schaftkopf einer endoskopischen Sonde übertragen werden.

Der Aktuator ist damit vergleichbar zu einem Bowdenzug aufgebaut, bei dem ebenfalls eine Bewegung eines in einer druckfesten Hülle geführten Drahtseils relativ zu der Hülle zur Übertragung einer mechanischen Bewegung sowie von Druck- und Zugkräften dient.

Der Aktuator ist dabei vorteilhafterweise so aufgebaut, dass er genau einen Formspeicher-Draht aufweist. Dieser besteht aus einem Stück oder ist aus mehreren in Längsrichtung aufeinanderfolgende Teilstücke zusammengesetzt. Der Formspeicher-Draht ist von der Draht-Isolierung in Längsrichtung allseitig umgeben und umschlossen. Davon ausgenommen ist gegebenenfalls allenfalls die elektrisch leitende Verbindung zu dem elektrischen Leiter, der den Formspeicher-Draht mit Strom versorgt. Die Draht-Isolierung ist von der Hülle umschlossen, so dass der von der Draht-Isolierung umgebene FormspeicherDraht in die Hülle eingebettet ist.

Der Formspeicher-Draht kann länger, kürzer oder gleich lang wie die Hülle sein.

Ein Ende oder beide Enden des mit der Draht-Isolierung ausgestatteten Formspeicher-Drahtes können innerhalb der Hülle angeordnet sein oder außerhalb der Hülle. Dies ist gegebenenfalls abhängig von dem Anschluss des Formspeicher-Drahtes an einen elektrischen Leiter und eine zugehörige Stromversorgung sowie. Ferner kann dies abhängig von der jeweiligen Anwendung sein.

Formgedächtnislegierungen sind spezielle Metalllegierungen, die in zwei unterschiedlichen Kristallstrukturen existieren können. Sie werden oft auch als Memorymetalle bezeichnet. Während die meisten Metalle immer dieselbe Kristallstruktur bis zu ihrem Schmelzpunkt besitzen, haben Formgedächtnislegierungen, abhängig von der Temperatur, zwei unterschiedliche Strukturen. Die Formumwandlung erfolgt aufgrund Änderung der Gitterstruktur in Abhängigkeit von der Temperatur. Es gibt in der Regel die Austenit genannte Hochtemperaturphase und die Martensit genannte Niedertemperaturphase. Beide können durch Temperaturänderung ineinander übergehen.

Die Strukturumwandlung ist unabhängig von der Geschwindigkeit der Temperaturänderung. Zur Einleitung der Phasenumwandlung sind die Parameter Temperatur und mechanische Spannung gleichwertig; das heißt, die Umwandlung kann nicht nur thermisch, sondern auch durch eine mechanische Spannung herbeigeführt werden.

Der Formspeicher-Draht wird zur Erzielung der gewünschten Bewegung durch elektrischen Strom beheizt. Dies erfolgt nicht durch eine zusätzliche Heizeinrichtung, insbesondere nicht durch zusätzliche Heizdrähte, sondern durch einen Strom, der durch den Formspeicher-Draht fließt. Die angelegte Spannung kann dabei je nach Anwendung Wechselspannung, konstante oder gepulste Gleichspannung gegebenenfalls in gezielter Form mit Puls-Weiten-Modulation sein. Die Erwärmung erfolgt dank des spezifischen Widerstands des leitenden Materials der Formgedächtnislegierung mit Hilfe der angelegten elektrischen Spannung, die über den Formspeicher-Draht in Form von Wärme abfällt. Der Draht befindet sich in einer elektrisch isolierenden und weitgehend thermisch stabilen Draht-Isolierung, welche wiederum zur bestmöglichen Krafterzeugung in einer Hülle gelagert ist.

Der Aktuator benötigt für seine Betätigung lediglich eine Spannungsquelle, mit der er über den elektrischen Leiter verbunden ist. Der Strom fließt über den elektrischen Leiter von der Spannungsquelle zum Formspeicher-Draht. Druckfluide werden nicht benötigt. Da der Formspeicher-Draht direkt durch den Strom erwärmt wird, der durch ihn fließt, muss nicht die gesamte Umgebung des Formspeicher-Drahtes erwärmt werden. Dies hat zur Folge, dass der Aktuator sich insgesamt weniger stark erwärmt als bekannte Aktuatoren mit Formgedächtnislegierungen, die mit zusätzlichen Heizeinrichtungen ausgestattet sind. Darüber hinaus ist der Formspeicher-Draht von einer Draht-Isolierung und von einer Hülle umgeben, die das Abstrahlen von Wärme reduzieren. Auf eine Kühlvorrichtung kann daher verzichtet werden. Der Aktuator weist einen kleinen Querschnitt auf und benötigt zu seiner Versorgung lediglich eine Stromzufuhr. Er ist daher bei Sonden mit kleinem Durchmesser und mit großem Durchmesser einsetzbar. Ferner ist die Sondenlänge durch den Aktuator nicht begrenzt, da außer einer Stromzufuhr für die Betätigung des Aktuators keine weiteren Einrichtungen benötigt werden. Eine entsprechende Stromzufuhr kann mittels des elektrischen Leiters über lange Distanzen zur Verfügung gestellt werden.

Nach einer vorteilhaften Ausgestaltung der Erfindung ist die Längenänderung des Formspeicher-Drahtes unter Einfluss der Wärme, welche durch den durch ihn fließenden Strom entsteht, eine Kontraktion. Der Formspeicher-Draht zieht sich zusammen, wenn durch ihn ein Strom fließt und er dabei auf eine vorgegebene Temperatur erwärmt wird. Dieses Zusammenziehen des Formspeicher-Drahtes führt zu einer Verkürzung der Länge des FormspeicherDrahtes von einer ersten Länge, die auch als Ausgangslänge bezeichnet werden kann, auf eine zweite Länge, die als Herstellungslänge bezeichnet werden kann. Diese Längenverkürzung kann in eine Zugkraft umgesetzt werden. Als Formgedächtnislegierung für den Formspeicher-Draht eignet sich insbesondere ein Material, bei dem der Formspeicher-Draht vor seinem Einsatz in den Aktuator mechanisch in die Länge gezogen wird. Dabei geht die durch die Herstellung des Formspeicher-Drahtes vorgegebene Herstellungslänge in die Ausgangslänge des Formspeicher-Drahtes über, wobei die Herstellungslänge kleiner ist als die Ausgangslänge. Unter der Einwirkung der Wärme, die sich bei dem stromdurchflossenen Formspeicher-Draht entwickelt, verkürzt sich der Formspeicher-Draht wieder und geht in seine Herstellungslänge über. Hierzu muss eine für die entsprechende Verformung notwendige Verformungstemperatur überschritten werden. Die Ausgangslänge entspricht dabei der in Anspruch 1 angegebenen ersten Länge. Die Herstellungslänge entspricht der in Anspruch angegebenen zweiten Länge. Wenn der Formspeicher-Draht nicht mehr von Strom durchflossen wird und unter die Verformungstemperatur abkühlt, kann er erneut mechanisch in die Länge gezogen werden, so dass er seine Herstellungslänge wieder einnimmt. Die hierfür notwendige Zugkraft kann entweder von einer Feder oder von einem zweiten Aktuator ausgebracht werden. Je zwei Aktuatoren können ein Aktuator-Paar bilden mit einem ersten Aktuator und einem zweiten Aktuator. Die beiden Aktuatoren werden abwechselnd mit Strom versorgt um einen Schaftkopf einer endoskopischen Sonde in die Richtung oder in die Gegenrichtung auszulenken. Dabei wird die Zugkraft des ersten Aktuators genutzt, um den FormspeicherDraht des zweiten Aktuators wieder in die Herstellungslänge zu dehnen. Entsprechend wird die Zugkraft des zweiten Aktuators genutzt, um den Formspeicher-Draht des ersten Aktuators wieder in die Herstellungslänge zu dehnen.

Nach einer weiteren vorteilhaften Ausgestaltung der Erfindung bildet die Draht-Isolierung ein flexibles Gleitrohr zwischen dem Formspeicher-Draht und der Hülle. Die Draht-Isolierung sorgt damit nicht nur für eine elektrische Isolierung des Formspeicher-Drahtes gegenüber seiner Umgebung, sondern auch für ein möglichst reibungsarmes Gleiten des Formspeicher-Drahtes in der Hülle, wenn der Formspeicher-Draht sich gegenüber der Hülle zusammenzieht oder wieder ausdehnt.

Nach einer weiteren vorteilhaften Ausgestaltung der Erfindung besteht die Draht-Isolierung aus Polyimid. Dieses Material ist elektrisch isolierend, thermisch stabil in dem vorgegebenen Temperatur-Bereich, innerhalb dem eine Verformung des Formspeicher-Drahtes bei der Betätigung des Aktuators stattfindet und weist darüber hinaus gute Gleiteigenschaften auf.

Nach einer weiteren vorteilhaften Ausgestaltung der Erfindung ist die Hülle durch ein Band oder einen Draht gebildet, der zu einer zylindrischen Spirale gewickelt ist. Eine derartige Spiral-Hülle weist die notwendigen Voraussetzungen einer Druckfestigkeit in Längsrichtung und einer Flexibilität in radialer Richtung auf, sie ist leicht herzustellenden und weist ein geringes Gewicht auf.

Nach einer weiteren vorteilhaften Ausgestaltung der Erfindung ist die Spirale aus Metall gewickelt. Hierzu eignet sich beispielsweise ein Band aus Federstahl, Edelstahl oder aus anderen Metallen. Die Spirale kann auch aus einem Draht gewickelt sein.

Nach einer weiteren vorteilhaften Ausgestaltung der Erfindung ist die Hülle mit einer die Hülle umgebenden Hüllen-Isolierung ausgestattet, welche die Hülle gegen die Umgebung thermisch isoliert. Damit wird eine Wärmeabstrahlung des Aktuators aufgrund des stromdurchflossenen Formspeicher-Drahts in die Umgebung des Aktuators.

Nach einer weiteren vorteilhaften Ausgestaltung der Erfindung ist ein Ende des Formspeicher-Drahtes an einem Gegenhalter mechanisch festgelegt. Damit ist vorgegeben, dass sich eine durch die Erwärmung des Formspeicher-Drahtes ausgelöste Längenänderung nur am entgegengesetzten Ende des Formspeicher-Drahtes bemerkbar macht. Bevorzugt ist die Hülle mit ihren einen Ende ebenfalls an dem Gegenhalter befestigt. Der Gegenhalter kann eine erste Gegenhalter-Komponente zur Festlegung des Formspeicher-Drahtes und eine zweite Gegenhalter-Komponente zur Festlegung der Hülle aufweisen.

Nach einer weiteren vorteilhaften Ausgestaltung der Erfindung ist der Formspeicher-Draht an dem Gegenhalter mit dem elektrischen Leiter verbunden. Da sich das an dem Gegenhalter befestigte Ende des Formspeicher-Drahtes nicht bewegt, ist es für die Verbindung mit dem elektrischen Leiter besonders gut geeignet. Insbesondere kann eine erste Gegenhalter-Komponente, welche ein Ende des Formspeicher-Drahtes festlegt, ausgebildet sein, um eine elektrisch leitende Verbindung zwischen dem elektrischen Leiter und dem FormspeicherDraht herzustellen. Ist ein Ende der Hülle an einer zweiten Gegenhalter-Komponente festgelegt, so ist die zweite Gegenhalter-Komponente gegen die erste Gegenhalter-Komponente elektrisch isoliert.

Nach einer weiteren vorteilhaften Ausgestaltung der Erfindung ist an das dem Gegenhalter abgewandte Ende des Formspeicher-Drahtes ein mechanisches Zugseil gekoppelt. Das Zugseil stellt in diesem Fall das Verbindungsstück zwischen dem Formspeicher-Draht des Aktuators und einem zu betätigenden Schaftkopf einer endoskopischen Sonde dar. Mit dem Zugseil kann eine Distanz zwischen dem Formspeicher-Draht und einem Schaftkopf einer endoskopischen Sonde überbrückt werden, innerhalb der keinerlei Erwärmung erfolgen soll oder innerhalb der kein Strom fließen soll.

Nach einer weiteren vorteilhaften Ausgestaltung der Erfindung ist der Formspeicher-Draht mit dem Zugseil über eine Hülse verbunden. Alternativ dazu können der Formspeicher-Draht und das Zugseil miteinander durch Kleben, Löten, Quetschen oder Schweißen verbunden sein.

Nach einer weiteren vorteilhaften Ausgestaltung der Erfindung ist das Zugseil zumindest abschnittsweise ebenfalls von der Draht-Isolierung und der Hülle umgeben. Alternativ kann das Zugseil auch nur abschnittsweise von der Draht-Isolierung umgeben sein. Als weitere Alternative kann das Zugseil auch nur abschnittsweise von der Hülle umgeben sein.

Nach einer weiteren vorteilhaften Ausgestaltung der Erfindung erstreckt sich durch Hülle über die im wesentlichen gesamte axiale Länge des FormspeicherDrahtes und über die im wesentlichen gesamte axiale Länge des Zugseils. Nach einer weiteren vorteilhaften Ausgestaltung der Erfindung ist der Formspeicher-Draht an einem Ende mit dem elektrischen Leiter verbunden und an dem anderen Ende an ein Bezugspotential, bevorzugt an ein Potential null, angeschlossen. Dieses Potential null wird auch als Masse bezeichnet.

Die endoskopische Sonde mit den Merkmalen des Anspruchs 1 zeichnet sich aus durch einen länglichen Schaft, welcher als Hohlkörper ausgebildet ist, durch einen Schaftkopf am distalen Ende des Schaftes, wobei der Schaftkopf gegenüber dem Schaft beweglich ist, und durch mindestens zwei erfindungsgemäßen Aktuator aus, welche den Schaftkopf relativ zu dem Schaft ausrichten.

Die Anzahl der eingesetzten Aktuatoren hängt von der Anwendung der endoskopischen Sonde ab. Ist nur eine Bewegung des Schaftkopfes relativ zum Schaft in eine Richtung notwendig, so können zwei Aktuatoren in die Sonde eingesetzt sein. Zur Bewegung des Schaftkopfes in einer dazu senkrechten Ebene kann die Sonde um die Längsachse des Schaftes gedreht werden. Bei einer Bewegung des Schaftkopfes in mindestens zwei Richtungen kann die Sonde mit drei oder vier Aktuatoren ausgestattet sein. Mit nur zwei Aktuatoren ist der Querschnitt des Schaftes kleiner als bei drei oder vier Aktuatoren. Eine Ausführungsform mit drei Aktuatoren weist den Vorteil auf, dass der Querschnitt des Schaftes kleiner ist als bei vier Aktuatoren und dennoch eine Bewegung des Schaftkopfes in mehrere Richtungen möglich ist. Bei einer Sonde mit drei Aktuatoren dienen bevorzugt zwei Aktuatoren dazu, den Schaftkopf nach einer Auslenkung durch den dritten Aktuator wieder in seine Ausgangsposition zu bewegen und gegebenenfalls den Formspeicher-Draht des dritten Aktuators wieder in seine Ausgangslänge zu ziehen. Hierzu müssen zwei Aktuatoren gleichzeitig angesteuert und mit Strom versorgt werden. Die Leistung der drei Aktuatoren kann hierbei je nach Ansteuerung verschieden sein.

Zur Bedienung der Aktuatoren der Sonde ist für einen Benutzer eine Bedieneinrichtung vorgesehen. Hierbei kann es sich um einen Steuerknüppel oder Steuerhebel, eine oder mehrere Schubregler oder eine Cursor-Steuerung für den Bildschirm eines Rechners handeln. Diese Bedieneinrichtung ist an eine Steuereinrichtung gekoppelt, die die Aktuatoren gezielt mit dem Strom einer Spannungsquelle versorgt.

Erfindungsgemäß sind die Aktuatoren in dem Schaft der endoskopischen Sonde angeordnet. Die durch den Formspeicher-Draht ausgeübte Zugkraft wird auf den Schaftkopf übertragen. Hierzu ist der Formspeicher-Draht direkt oder indirekt mit dem Schaftkopf verbunden.

Nach einer weiteren vorteilhaften Ausgestaltung der Erfindung ist der Aktuator mit seiner Längsachse im wesentlichen parallel zur Längsachse des Schaftes ausgerichtet.

Erfindungsgemäß sind mindestens zwei Aktuatoren in dem Schaft der endoskopischen Sonde derart angeordnet, dass die Formspeicher-Drähte der Aktuatoren in axialer Richtung des Schaftes versetzt zueinander und frei von einer Überlappung in axialer Richtung sind. Dadurch wird ausgeschlossen, dass sich die Formspeicher-Drähte gegenseitig durch Wärmeabstrahlung erwärmen können. Eine derartige gegenseitige Erwärmung kann zu einer Verfälschung der Bewegung der Aktuatoren führen. Eine gegenseitige thermische Beeinflussung oder sonstige Beeinträchtigung der Aktuatoren untereinander wird durch die versetzte Anordnung ausgeschlossen.

Nach einer weiteren vorteilhaften Ausgestaltung der Erfindung ist die endoskopische Sonde mit einer Spannungsversorgung ausgestattet. Ferner ist die endoskopische Sonde mit einer Steuereinrichtung ausgestattet, welche den mindestens einen Aktuator zur Bewegung des Schaftkopfes derart mit Strom versorgt, dass der Aktuator den Schaftkopf aus einer vorgegebenen Ausgangsposition ausgelenkt und in eine vorgegebene Endposition bewegt und den Schaftkopf aus der Endposition wieder in seine Ausgangsposition bewegt. Die Bewegung des Schaftkopfes erfolgt anhand der Steuereinrichtung entsprechend den von einem Benutzer der endoskopischen Sonde vorgegebenen Vorgaben. Hierzu kann eine Bedieneinrichtung vorgesehen sein. Derartige Bedieneinrichtungen für einen Benutzer sind zur Betätigung von endoskopischen Sonden aus dem Stand der Technik bekannt.

Nach einer weiteren vorteilhaften Ausgestaltung der Erfindung ist die Steuereinrichtung ausgebildet, den Widerstand des Formspeicher-Drahtes des Aktuators oder der Aktuatoren zu bestimmen und aus dem Widerstand den Ist-Zustand der Auslenkung des Formspeicher-Drahtes zu bestimmen, um diesen mit einem vorgegebenen Soll-Zustand der Auslenkung des FormspeicherDrahtes abzugleichen. Der Formspeicher-Draht muss nicht dauerhaft bestromt werden. In regelmäßigen kurzen Pausen der Bestromung von wenigen Teilen einer Sekunde kann der elektrische Widerstand ausgemessen und gleichzeitig die Position des Aktuators erfasst werden, um eine Überbelastung des Aktuators und der angeschlossenen Mechanik zu vermeiden.

Nach einer weiteren vorteilhaften Ausgestaltung der Erfindung ist die endoskopische Sonde mit mindestens einem Temperatursensor ausgestattet. Die von dem Temperatursensor erfasste Temperatur wird an die Steuereinrichtung ausgegeben. Anhand der vorgegebenen Widerstandskennlinie kann von der Temperatur des Formspeicher-Drahtes auf seinen spezifischen Widerstand geschlossen werden. Dieser Widerstand kann mit einem eventuell gemessenen Widerstand verglichen werden. Anhand von Temperatur und Widerstand kann der Aktuator mittels der Steuereinrichtung gesteuert werden. Der Ist-Zustand des Aktuators kann erfasst und mit einem Soll-Zustand verglichen werden.

Nach einer weiteren vorteilhaften Ausgestaltung der Erfindung ist die endoskopische Sonde mit mindestens einem ersten Temperatursensor ausgestattet, der die Temperatur an dem Formspeicher-Draht des Aktuators oder der Aktuatoren bestimmt. Auf dieser Weise kann erfasst werden, ob die an dem Formspeicher-Draht herrschende Temperatur mit der durch den fließenden Strom und den Widerstand vergebenen Temperatur übereinstimmt. Einflüsse durch Wärmeleitung oder Wärmestrahlung aus der Umgebung des Formspeicher-Drahtes können auf diese Weise erfasst werden und von der Steuereinrichtung berücksichtigt werden.

Nach einer weiteren vorteilhaften Ausgestaltung der Erfindung ist die endoskopische Sonde mit einem zweiten Temperatursensor ausgestattet, der die Umgebungstemperatur des Schaftes oder des Schaftkopfes bestimmt. Auch auf diese Weise kann ein Einfluss einer Wärmequelle in der Umgebung des Formspeicher-Drahtes erfasst und gegebenenfalls eliminiert werden.

Nach einer weiteren vorteilhaften Ausgestaltung der Erfindung ist in dem Schaft genau ein Aktuator angeordnet, der den Schaftkopf aus seiner Ausgangsposition in eine Endposition bewegt. Um den Schaftkopf wieder in seine Ausgangsposition zurück zu bewegen, ist die endoskopische Sonde mit einer Feder ausgestattet. Der Schaftkopf kann in diesem Fall nur in eine Richtung ausgelenkt und wieder in seine Ausgangsposition zurückbewegt werden. Um eine Auslenkung des Schaftkopfes in eine andere Richtung zu ermöglichen, kann die endoskopische Sonde insgesamt um die Längsachse des Schaftes gedreht werden.

Nach einer weiteren vorteilhaften Ausgestaltung der Erfindung ist die endoskopische Sonde mit einer geradzahligen Anzahl an Aktuatoren ausgestattet. Jeweils zwei Aktuatoren bilden ein Aktuatoren-Paar mit einem ersten Aktuator und einem zweiten Aktuator. Wird der Schaftkopf durch den ersten Aktuator ausgelenkt und von seiner Ausgangsposition in eine Endposition bewegt, so sorgt der zweite Aktuator dafür, dass der Schaftkopf anschließend wieder in die Ausgangsposition zurück bewegt wird. Entsprechendes gilt bei einer Auslenkung des Schaftkopfes durch den zweiten Aktuator. Der erste und der zweite Aktuator bilden dabei Gegenspieler.

Nach einer weiteren vorteilhaften Ausgestaltung der Erfindung weist der Schaft einen länglichen Querschnitt auf. Der Querschnitt kann insbesondere rechteckig sein. Der längliche Schaft weist dabei die Form eines Schwertes oder eine Lanze auf.

Nach einer weiteren vorteilhaften Ausgestaltung der Erfindung ist der Schaftkopf gegenüber dem Schaft um eine Schaftkopf-Drehachse drehbar, die zu der Längsachse des Schaftes im wesentlichen senkrecht ausgerichtet ist.

Nach einer weiteren vorteilhaften Ausgestaltung der Erfindung ist der Schaftkopf als Drehteller ausgebildet. Ist der als Drehteller ausgebildete Schaftkopf mit einem Schaft in Form eines Schwertes oder eine Lanze kombiniert und ist der Drehteller parallel zu dem Schwert- oder Lanzen-förmigen Schaft ausgerichtet, so weist die Sonde insgesamt eine flache Form auf. Derartige Sonden eignen sich beispielsweise für die Prüfung und Untersuchung von Turbinen und von Rohrbündeln in Wärmetauschern. Derartige Sonden können beispielsweise von einem Roboter oder Manipulator reproduzierbar an dem vorgesehenen Einsatzort in einen Hohlraum einführt werden. Dabei kann die Sonde für die Einführung in den Hohlraum aufgewickelt werden. Während bekannte Sonden die aufgewickelte Lagerung der Sonde in der Regel stark in der Bewegung gehemmt sind, stellt dies bei der erfindungsgemäßen Sonde keine Beeinträchtigung dar. Die erfindungsgemäße Sonde unterscheidet sich von bekannten Sonden ferner dadurch, dass nur der Schaftkopf gegenüber dem Schaft bewegt wird und nicht der gesamte Schaft abgewinkelt wird

Nach einer weiteren vorteilhaften Ausgestaltung der Erfindung ist der Formspeicher-Draht direkt an dem Schaftkopf befestigt.

Nach einer weiteren vorteilhaften Ausgestaltung der Erfindung der Schaftkopf mehrere relativ zueinander bewegliche ring- oder rohrförmige Schaftkopfsegmente auf, welche durch den oder die Aktuatoren relativ zueinander ausgerichtet werden. Hierzu können die Schaftkopfsegmente Durchgangsöffnungen aufweisen, durch die die Formspeicher-Drähte der Aktuatoren oder daran gekoppelte Zugseile hindurchgeführt sind.

Nach einer weiteren vorteilhaften Ausgestaltung der Erfindung bestehen die Schaftkopfsegmente aus Kunststoff oder aus einem anderen elektrischen Isolator.

Nach einer weiteren vorteilhaften Ausgestaltung der Erfindung weisen die Schaftkopfsegmente Durchgangsöffnungen auf, durch die die Formspeicher-Drähte oder an die Formspeicher-Drähte der Aktuatoren gekoppelte Zugseile hindurchgeführt sind.

Das Verfahren zur Steuerung der Aktuatoren einer endoskopischen Sonde, das nicht Teil der vorliegenden Erfindung ist, zeichnet sich dadurch aus, dass die Temperatur an dem Aktuator erfasst wird, dass der Widerstand des Formspeicher-Drahtes des Aktuators erfasst wird und dass der Strom, mit dem der Formspeicher-Draht versorgt wird, in Abhängigkeit von der Temperatur und dem Widerstand gesteuert wird.

Weitere Vorteile und vorteilhafte Ausgestaltungen der Erfindung sind der nachfolgenden Beschreibung, der Zeichnung und den Ansprüchen entnehmbar.

### Zeichnung

In der Zeichnung sind Ausführungsbeispiel eines erfindungsgemäßen Aktuators und zweier erfindungsgemäßer endoskopischer Sonden dargestellt. Es zeigen:
- Figur 1: Ausschnitt aus einem Aktuator in Seitenansicht,
- Figur 2: Gegenhalter und elektrischer Leiter des Aktuators gemäß Figur 1,
- Figur 3: erstes Ausführungsbeispiel einer endoskopischen Sonde,
- Figur 4: Teil des Schaftes mit Schaftkopf der Sonde gemäß Figur 3,
- Figur 5: zweites Ausführungsbeispiel einer endoskopischen Sonde,
- Figur 6: Teil des Schaftes mit Schaftkopf der Sonde gemäß Figur 5,
- Figur 7: Sonde gemäß Figur 5 mit einem umgebenden Schlauch.

### Beschreibung der Ausführungsbeispiele

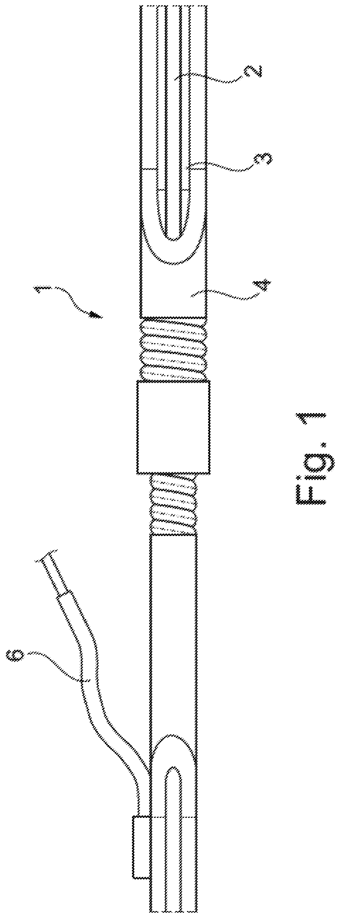

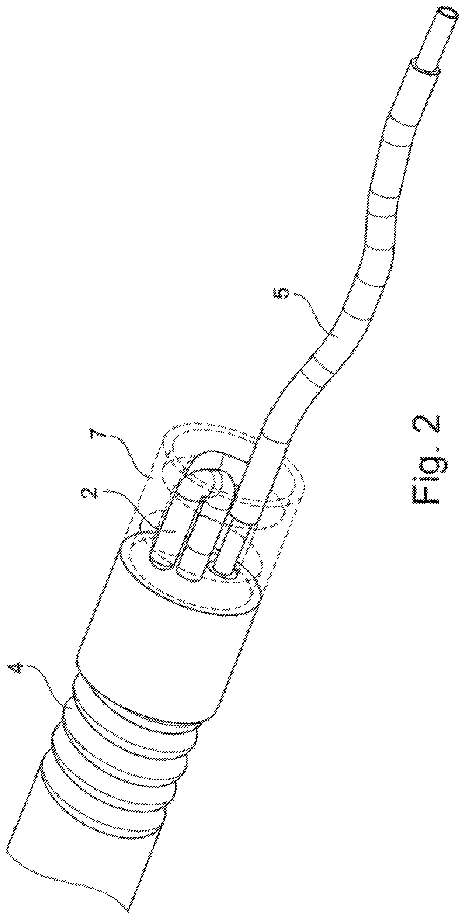

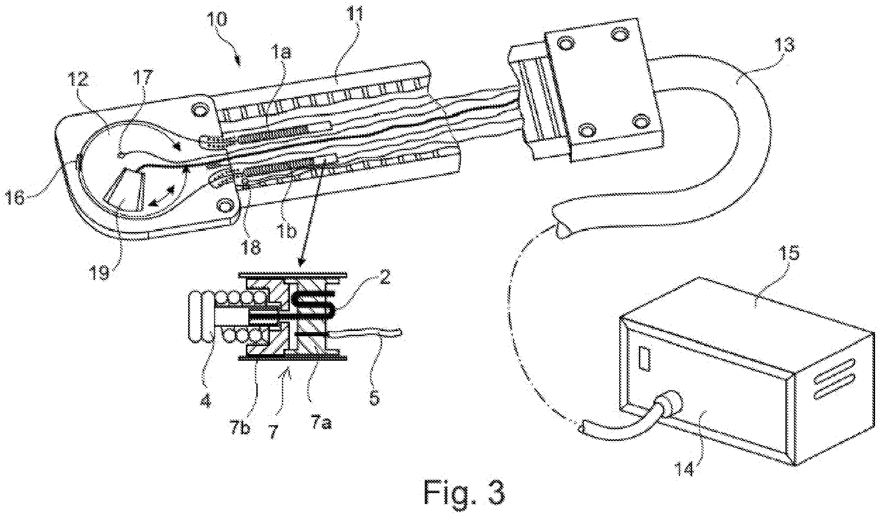

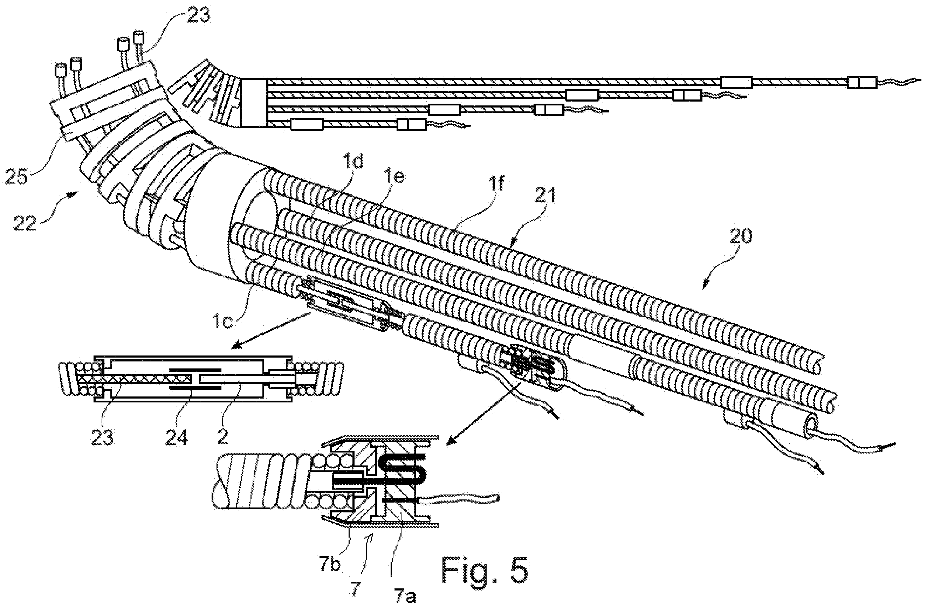

In den Figuren 1 und 2 ist ein Ausführungsbeispiel eines Aktuators 1 in Seitenansicht dargestellt. Der Aktuator 1 weist einen Formspeicher-Draht 2, eine Draht-Isolierung 3, eine Hülle 4, einen ersten elektrischen Leiter 5, welcher mit einer nicht dargestellten Spannungsquelle verbunden werden kann und den Formspeicher-Draht 2 mit Strom versorgt, und einen zweiten elektrischen Leiter 6, welcher den Formspeicher-Draht 2 mit Potential null verbindet. Der Formspeicher-Draht 2 ist von der Draht-Isolierung 3 umgeben, welche ihrerseits von der Hülle 4 umgeben ist. Die Hülle 4 besteht aus einem Federstahl-Band, welches zu einer zylindrischen Spirale gewickelt ist. Die Hülle 4 ist in Figur 1 nur abschnittsweise dargestellt. Sie erstreckt sich jedoch tatsächlich von einem in Figur 2 dargestellten Gegenhalter 7 bis zu dem entgegengesetzten Ende des Aktuators, welches in den Figuren 1 und 2 nicht dargestellt ist. An dem Gegenhalter 7 sind der Formspeicher-Draht 2 und die Hülle 4 befestigt. Hierzu ist der Formspeicher-Draht 2 durch mehrere Durchgangsöffnungen des Gegenhalters 7 hindurchgeführt. Der Abschnitt des Gegenhalters 7, der die Durchgangsöffnungen aufweist, ist in Figur 2 durchsichtig dargestellt. Der Gegenhalter 7 besteht abschnittsweise aus einem elektrisch leitfähigen Material. In dem Gegenhalter 7 ist der erste elektrische Leiter 5 derart angeordnet, dass der elektrische Strom von dem ersten elektrischen Leiter 5 zu dem FormspeicherDraht 2 fließen kann. Über den zweiten elektrischen Leiter 6 kann der Strom aus dem Formspeicher-Draht 2 abfließen. Der Abschnitt des Gegenhalters 7, der mit der Hülle 4 verbunden ist, ist gegen elektrisch leitfähigen Abschnitt des Gegenhalters 7 elektrisch isoliert. Der Gegenhalter 7 weist hierzu eine erste Gegenhalter-Komponente 7a zur Aufnahme des Formspeicher-Drahtes 2 und eine zweite Gegenhalter-Komponente zur Aufnahme der Hülle 4 auf. Diese beiden Gegenhalter-Komponenten 7a, 7b sind in den Figuren 3 und 5 dargestellt.

Das dem Gegenhalter 7 abgewandte Ende des Formspeicher-Drahtes 2, welches in den Figuren 1 und 2 nicht dargestellt ist, ragt entweder aus der Hülle 4 heraus oder der Formspeicher-Draht 2 ist noch innerhalb der Draht-Isolierung 3 und der Hülle 4 mit einem nicht dargestellten Zugseil verbunden, das aus der Hülle 4 herausragt. Der aus der Hülle herausragende Teil wird mit einem zu bewegenden Schaftkopf einer endoskopischen Sonde verbunden. Ausführungsbeispiele hierzu sind in den Figuren 3 bis 7 dargestellt.

Fließt durch den Formspeicher-Draht 2 ein elektrischer Strom, welcher durch den ersten elektrischen Leiter 5 zugeführt und durch den zweiten elektrischen Leiter abgeleitet wird, so erwärmt sich der Formspeicher-Draht 2. Aufgrund dieser Erwärmung erfolgt eine Formwandlung der Formgedächtnislegierung des Formspeicher-Drahtes basierend auf einer temperaturabhängigen Gitterumwandlung zu einer der beiden Kristallstrukturen der Formgedächtnislegierung. Dies führt dazu, dass sich die Länge des Formspeicher-Drahtes verkürzt. Da das eine Ende des Formspeicher-Drahtes 2 an dem Gegenhalter 7 festgelegt ist und die Hülle 4 ebenfalls an dem Gegenhalter 7 befestigt ist und in ihrer axialen Länge konstant bleibt, bewegt sich das dem Gegenhalter abgewandte Ende des Formspeicher-Drahtes 2 relativ zu der Hülle 3. Diese Bewegung wird auf einen Schaftkopf übertragen. Auf den Schaftkopf wirkt dann eine Zugkraft.

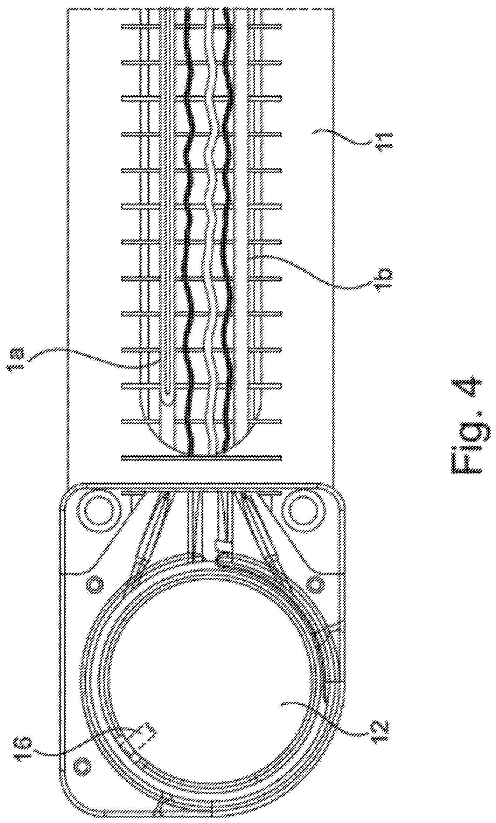

In den Figuren 3 und 4 ist ein erstes Ausführungsbeispiel einer endoskopischen Sonde 10 dargestellt. Die Sonde 10 umfasst einen länglichen Schaft 11 und einen gegenüber dem Schaft 11 beweglichen Schaftkopf 12. Der Schaft 11 ist über ein Kabel 13 mit einem Gehäuse verbunden, in welchem eine Spannungsversorgung 14 und eine Steuereinrichtung 15 aufgenommen sind. Der Schaft 11 weist einen rechteckigen Querschnitt auf, so dass er eine Schwert- oder Lanzenform annimmt. Die dem Betrachter in den Figuren 3 und 4 zugewandte Oberfläche des Schaftes 11 ist größer als die senkrecht dazu ausgerichtete Oberfläche des Schaftes 11. Der Schaftkopf 12 ist als Drehteller ausgebildet. Die Drehachse dieses Drehtellers verläuft senkrecht zur Zeichenebene in Figur 3 und 4 und damit senkrecht zur Längsachse des Schaftes 11. In dem Schaft 11 sind zwei Aktuatoren 1a, 1b angeordnet, die mit dem Aktuator gemäß Figuren 1 und 2 übereinstimmen. Für die einzelnen Komponenten der Aktuatoren sind daher in den Figuren 3 und 4 die entsprechenden Bezugszahlen verwendet wie in den Figuren 1 und 2. Der Formspeicher-Draht 2 der beiden Aktuatoren 1a, 1b ragt mit seinem dem Gegenhalter 7 abgewandten Ende aus der Hülle 4 heraus. Der Gegenhalter 7 weist eine erste Gegenhalter-Komponente 7a auf, in welchem der Formspeicher-Draht 2 mit einem Ende festgelegt ist. In der ersten Gegenhalter-Komponente 7a ist ferner der erste elektrische Leiter 5 aufgenommen, so dass der erste elektrische Leiter mit dem Formspeicher-Draht elektrisch leitend verbunden ist. Ferner weist der Gegenhalter 7 eine zweite Gegenhalter-Komponente 7b auf, in welchem die Hülle 4 mit einem Ende festgelegt ist. Die beiden Gegenhalter-Komponenten 7a und 7b sind derart ausgebildet, dass die Hülle 4 gegen den ersten elektrischen Leiter 5 und den Formspeicher-Draht 2 elektrisch isoliert ist.

Das dem Gegenhalter 7 abgewandte, distale Ende des Formspeicher-Drahtes 2 ist um einen Teil des Umfangs des als Drehteller ausgebildeten Schaftkopfes 12 herumgeführt und mittels einer Schraube 16 an dem Schaftkopf 12 befestigt. Eine Erwärmung des Formspeicher-Drahtes des Aktuators 1a führt daher zu einer Drehung des Schaftkopfes im Uhrzeigersinn. Eine Erwärmung des Formspeicher-Drahtes des Aktuators 1b führt zu einer Drehung entgegen des Uhrzeigersinns. Die beiden Drehrichtungen sind in Figur 3 durch Pfeile angedeutet. Mittels eines ersten Temperatursensors 17 und eines zweiten Temperatursensors 18 wird die Temperatur am Schaftkopf 12 und am Schaft 11 erfasst und an die Steuereinrichtung 15 ausgegeben. Hierzu sind die beiden Temperatursensoren mit der Steuereinrichtung 15 verbunden. Die Steuereinrichtung berücksichtigt die erfasste Temperatur bei der Vorgabe des Stroms, mit dem die Aktuatoren versorgt werden.

Auf dem Schaftkopf 12 ist eine Kamera 19 angeordnet.

In den Figuren 5, 6 und 7 ist ein zweites Ausführungsbeispiel einer endoskopischen Sonde 20 dargestellt. Figur 5 zeigt die Sonde 20 in verschiedenen Ansichten. Die Sonde 20 weist einen länglichen Schaft 21 und einen Schaftkopf 22 auf. Der Schaft ist mit insgesamt vier Aktuatoren 1c, 1d, 1e, 1f ausgestattet, die mit dem Aktuator gemäß Figuren 1 und 2 übereinstimmen. Die Aktuatoren sind in dem Schaft 21 in axialer Richtung derart angeordnet, so dass sich die Formspeicher-Drähte in axialer Richtung nicht überlappen. Sie sind versetzt zueinander. Dies ist in dem oberen Teil der Figur 5 dargestellt. Die axiale Länge der Aktuatoren 1c, 1d, 1e, 1f ist unterschiedlich. Der Aktuator 1c weist die kleinste Länge in axialer Richtung auf, der Aktuator 1f die größte. An den distalen Enden sind die Formspeicher-Drähte 2 mit Zugseilen 23 über eine Hülse 24 verbunden. Dies ist in einem Ausschnitt des Aktuators 1c dargestellt. Der Schaftkopf weist mehrere ringförmige Schaftkopfsegmente 25 auf. Je zwei Aktuatoren 1c, 1d und 1e, 1f bilden ein Aktuatoren-Paar. In einem Aktuatoren-Paar wird die Bewegungen des jeweils einen Aktuators durch Bewegung des jeweils anderen Aktuators kompensiert, so dass der Schaftkopf 22 in seine Ausgangsposition zurück kehrt.

Die ringförmigen Schaftkopfsegmente 25 sind mit mehreren Durchgangsöffnungen ausgestattet, durch welche die Zugseile 23 hindurchgeführt sind. Eine Bewegung der Aktuatoren 1c, 1d, 1e, 1f führt dazu, dass die Schaftkopfsegmente 25 relativ zueinander ausgerichtet werden.

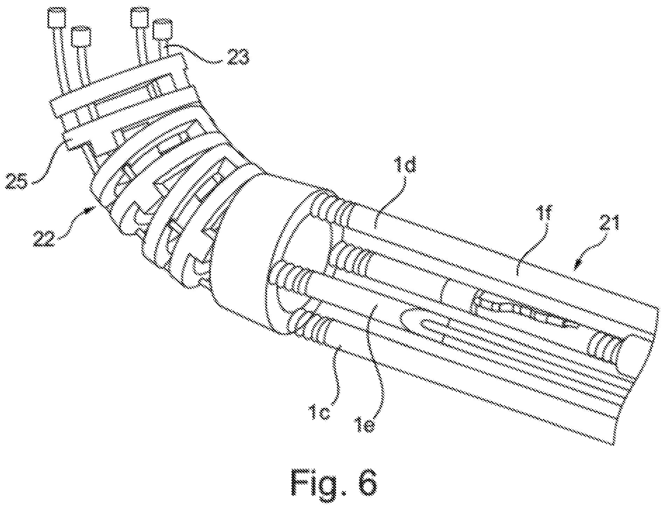

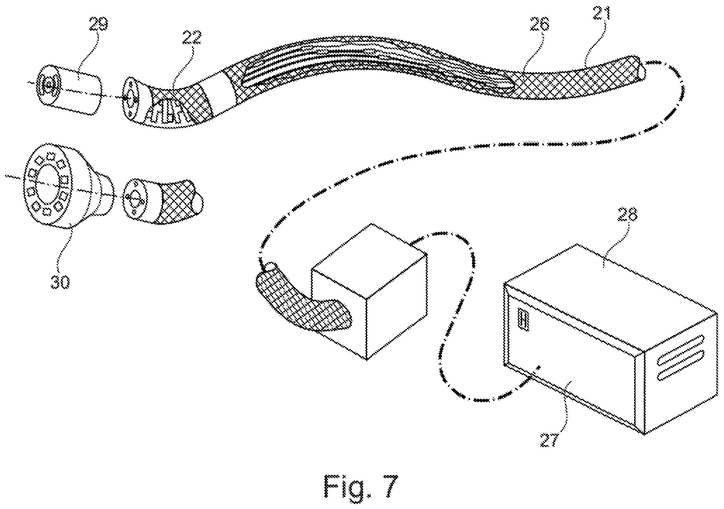

Figur 7 zeigt die endoskopische Sonde gemäß Figuren 5 und 6 mit einem Schlauch 26, der die gesamte Sonde 20 umgibt und mit einer kombinierten Spannungsversorgung 27 und Steuereinrichtung 28. Die Sonde weist dabei eine erhebliche axiale Länge auf. Auf den Schaftkopf 22 kann eine Kamera 29 oder 30 ausgesteckt werden.

Sämtliche Merkmale können sowohl einzeln als auch in beliebiger Kombination miteinander erfindungswesentlich sein.

### Bezugszahlen

- 1: Aktuator
- 1a: Aktuator
- 1b: Aktuator
- 1c: Aktuator
- 1d: Aktuator
- 1e: Aktuator
- 1f: Aktuator
- 2: Formspeicher-Draht
- 3: Draht-Isolierung
- 4: Hülle
- 5: Erster elektrischer Leiter
- 6: Zweiter elektrischer Leiter
- 7: Gegenhalter
- 7a: erste Gegenhalter-Komponente
- 7b: zweite Gegenhalter-Komponente
- 8 9 10: Endoskopische Sonde
- 11: Schaft
- 12: Schaftkopf
- 13: Kabel
- 14: Spannungsversorgung
- 15: Steuereinrichtung
- 16: Schraube
- 17: Erster Temperatursensor
- 18: Zweiter Temperatursensor
- 19: Kamera
- 20: Endoskopische Sonde
- 21: Schaft
- 22: Schaftkopf
- 23: Zugseil
- 24: Hülse
- 25: Schaftkopfsegment
- 26: Schlauch
- 27: Spannungsversorgung
- 28: Steuereinrichtung
- 29: Kamera
- 30: Kamera

## Patentansprüche

1. Endoskopische Sonde
mit einem länglichen Schaft (11, 21), welcher als Hohlkörper ausgebildet ist,
mit einem proximalen Ende des Schaftes (11, 21),
mit einem distalen Ende des Schaftes (11, 21),
mit einem Schaftkopf (12, 22) am distalen Ende des Schaftes (11, 21), wobei der Schaftkopf (12, 22) gegenüber dem Schaft (11, 21) beweglich ist,
mit mindestens zwei Aktuatoren, welche dazu eingerichtet sind, den Schaftkopf (12, 22) relativ zum Schaft (11, 21) auszurichten,
wobei jeder Aktuator ausgestattet ist
mit einem länglichen Formspeicher-Draht (2) aus einer Formgedächtnislegierung,
mit einem elektrischen Leiter (5), der elektrisch leitend mit dem Formspeicher-Draht (2) verbunden ist und dazu eingerichtet ist, den Formspeicher-Draht (2) mit Strom zu versorgen,
wobei der Formspeicher-Draht (2) ausgebildet ist, die elektrische Energie des durch ihn fließenden Stroms in Wärmeenergie umzuwandeln und seine Länge unter Einfluss dieser Wärme ausgehend von einer ersten Länge in eine zweite Länge zu ändern,
mit einer den Formspeicher-Draht (2) zumindest abschnittweise umgebenden Draht-Isolierung (3) aus einem elektrisch isolierenden Material,
mit einer flexiblen und in Längsrichtung des Formspeicher-Drahtes (2) druckfesten Hülle (3), welche eine Führung für den Formspeicher-Draht (2) und ein Gegenlager zur Abstützung der zu übertragenden, durch die Formänderung des Formspeicher-Drahtes (2) wirkenden Zugkräfte bildet, und welche die Draht-Isolierung (3) umgibt
**dadurch gekennzeichnet,**
**dass** die mindestens zwei Aktuatoren (1, 1a, 1b, 1c, 1d, 1e, 1f) in dem Schaft (11, 21) derart angeordnet sind, dass die Formspeicher-Drähte (2) der Aktuatoren (1, 1a, 1b, 1c, 1d, 1e, 1f) in axialer Richtung des Schaftes versetzt zueinander und frei von einer Überlappung in axialer Richtung sind.

2. Endoskopische Sonde nach Anspruch 1, **dadurch gekennzeichnet, dass** die Längenänderung des Formspeicher-Drahtes (2) unter Einfluss der Wärme, welche durch den durch ihn fließenden Strom entsteht, eine Kontraktion ist.

3. Endoskopische Sonde nach Anspruch 1 oder 2, **dadurch gekennzeichnet, dass** die Draht-Isolierung (3) in flexibles Gleitrohr zwischen dem Formspeicher-Draht (2) und der Hülle (4) bildet.

4. Endoskopische Sonde nach Anspruch 1, 2 oder 3, **dadurch gekennzeichnet, dass** die Draht-Isolierung (3) aus Polyimid besteht.

5. Endoskopische Sonde nach einem der vorhergehenden Ansprüche, **dadurch gekennzeichnet, dass** die Hülle (4) durch ein Band oder einen Draht gebildet ist, der zu einer zylindrischen Spirale gewickelt ist.

6. Endoskopische Sonde nach Anspruch 5, **dadurch gekennzeichnet, dass** die Spirale aus Metall gewickelt ist.

7. Endoskopische Sonde nach einem der vorhergehenden Ansprüche,
**dadurch gekennzeichnet, dass** die Hülle (4) mit einer die Hülle (4) umgebenden Hüllen-Isolierung ausgestattet ist, welche die Hülle (4) gegen die Umgebung thermisch isoliert.

8. Endoskopische Sonde nach einem der vorhergehenden Ansprüche,
**dadurch gekennzeichnet, dass** ein Ende des Formspeicher-Drahtes (2) an einem Gegenhalter (7, 7a) mechanisch festgelegt ist, und dass die Hülle (4) mit ihren einen Ende ebenfalls an dem Gegenhalter (7, 7b) befestigt ist.

9. Endoskopische Sonde nach Anspruch 8, **dadurch gekennzeichnet, dass** der Formspeicher-Draht (2) an dem Gegenhalter (7, 7a) mit dem elektrischen Leiter (5) verbunden ist.

10. Endoskopische Sonde nach Anspruch 8 oder 9, **dadurch gekennzeichnet, dass** an das dem Gegenhalter (7, 7a) abgewandte Ende des Formspeicher-Drahtes (2) ein mechanisches Zugseil (23) gekoppelt ist.

11. Endoskopische Sonde nach Anspruch 10, **dadurch gekennzeichnet, dass** der Formspeicher-Draht (2) mit dem Zugseil (23) über eine Hülse (24) verbunden ist.

12. Endoskopische Sonde nach Anspruch 10 oder 11, **dadurch gekennzeichnet, dass** das Zugseil (23) zumindest abschnittsweise ebenfalls von der Draht-Isolierung (3) und der Hülle (4) umgeben ist.

13. Endoskopische Sonde nach einem der vorhergehenden Ansprüche,
**dadurch gekennzeichnet, dass** der Formspeicher-Draht (2) an einem Ende mit dem elektrischen Leiter (5) verbunden ist und an dem anderen Ende an ein Bezugspotential gekoppelt ist.

14. Endoskopische Sonde nach Anspruch 1, **dadurch gekennzeichnet, dass** der Aktuator (1, 1a, 1b, 1c, 1d, 1e, 1f) mit seiner Längsachse im wesentlichen parallel zur Längsachse des Schaftes (11, 21) ausgerichtet ist.

15. Endoskopische Sonde nach einem der Ansprüche 1 bis 14, **dadurch gekennzeichnet, dass** sie mit einer Spannungsversorgung (14, 27) ausgestattet ist, und dass sie mit einer Steuereinrichtung (15, 28) ausgestattet ist, welche die Aktuatoren (1, 1a, 1b, 1c, 1d, 1e, 1f) zur Bewegung des Schaftkopfes (12, 22) derart mit Strom versorgt, dass die Aktuatoren (1, 1a, 1b, 1c, 1d, 1e, 1f) den Schaftkopf (12, 22) aus einer vorgegebenen Ausgangsposition auslenken und in eine vorgegebene Endposition bewegen, und den Schaftkopf (12, 22) aus der Endposition wieder in seine Ausgangsposition bewegen.

16. Endoskopische Sonde nach Anspruch 15, **dadurch gekennzeichnet, dass** die Steuereinrichtung (15, 28) ausgebildet ist, den Widerstand des Formspeicher-Drahtes (2) des Aktuators (1, 1a, 1b, 1c, 1d, 1e, 1f) oder der Aktuatoren (1, 1a, 1b, 1c, 1d, 1e, 1f) zu bestimmen und aus dem Widerstand den Ist-Zustand der Auslenkung des Formspeicher-Drahtes (2) zu bestimmen, um diesen mit einem vorgegebenen Soll-Zustand der Auslenkung des Formspeicher-Drahtes (2) abzugleichen.

17. Endoskopische Sonde nach einem der Ansprüche 1 bis 16, **dadurch gekennzeichnet, dass** sie mit mindestens einem Temperatursensor (17, 18) ausgestattet ist.

18. Endoskopische Sonde nach Anspruch 17, **dadurch gekennzeichnet, dass** sie mit mindestens einem ersten Temperatursensor (17) ausgestattet ist, der die Temperatur an dem Formspeicher-Draht (2) des Aktuators oder der Aktuatoren (1, 1a, 1b, 1c, 1d, 1e, 1f) bestimmt.

19. Endoskopische Sonde nach Anspruch 17 oder 18, **dadurch gekennzeichnet, dass** sie mit einem zweiten Temperatursensor (18) ausgestattet ist, der die Umgebungstemperatur des Schaftes (11, 21) oder des Schaftkopfes (12, 22) bestimmt.

20. Endoskopische Sonde nach einem der Ansprüche 15 bis 19, **dadurch gekennzeichnet, dass** sie mit einer geradzahligen Anzahl an Aktuatoren (1, 1a, 1b, 1c, 1d, 1e, 1f) ausgestattet ist, von denen jeweils zwei Aktuatoren (1, 1a, 1b, 1c, 1d, 1e, 1f) ein Aktuatoren-Paar mit einem ersten Aktuator und einem zweiten Aktuator bilden, wobei die beiden Aktuatoren (1, 1a, 1b, 1c, 1d, 1e, 1f) eines Aktuatoren-Paares ihre Formspeicher-Drähte (2) gegenseitig auf eine vorgegebene zweite Länge ausdehnen.

21. Endoskopische Sonde nach einem der Ansprüche 1 bis 20, **dadurch gekennzeichnet, dass** der Schaft (11) einen länglichen Querschnitt aufweist.

22. Endoskopische Sonde nach Anspruch 1 bis 21, **dadurch gekennzeichnet, dass** der Schaftkopf (12) gegenüber dem Schaft (11) um eine Schaftkopf-Drehachse drehbar ist, die zu der Längsachse des Schaftes (11) im wesentlichen senkrecht ausgerichtet ist.

23. Endoskopische Sonde nach Anspruch 22, **dadurch gekennzeichnet, dass** der Schaftkopf (12) als Drehteller ausgebildet ist.

24. Endoskopische Sonde nach einem der Ansprüche 1 bis 23, **dadurch gekennzeichnet, dass** der Formspeicher-Draht (2) des Aktuators (1, 1a, 1b, 1c, 1d, 1e, 1f) an dem Schaftkopf (12) direkt befestigt ist.

25. Endoskopische Sonde nach einem der Ansprüche 1 bis 21, **dadurch gekennzeichnet, dass** der Schaftkopf (22) mehrere relativ zueinander bewegliche ring- oder rohrförmige Schaftkopfsegmente (25) aufweist, welche durch den oder die Aktuatoren (1c, 1d, 1e, 1f) ausgerichtet werden.

26. Endoskopische Sonde nach Anspruch 25, **dadurch gekennzeichnet, dass** die Schaftkopfsegmente (25) aus Kunststoff bestehen.

27. Endoskopische Sonde nach Anspruch 25 oder 26, **dadurch gekennzeichnet, dass** die Schaftkopfsegmente (25) Durchgangsöffnungen aufweisen, durch die die Formspeicher-Drähte (2) oder an die Formspeicher-Drähte (2) der Aktuatoren (1c, 1d, 1e, 1f) gekoppelte Zugseile (23) hindurchgeführt sind.

## Claims

1. Endoscopic probe
with an elongated shaft (11, 21), which has the form of a hollow body,
with a proximal end of the shaft (11, 21),
with a distal end of the shaft (11, 21),
with a shaft head (12, 22) on the distal end of the shaft (11, 21), wherein the shaft head (12, 22) can be moved in relation to the shaft (11, 21),
with at least two actuators which are configured to align the shaft head (12, 22) relative to the shaft (11,21),
wherein each actuator is provided with an elongate shape memory wire (2) consisting of a shape memory alloy,
with an electrical conductor (5) which is electrically conductively connected to the shape memory wire (2) and configured to supply the shape memory wire (2) with current,
wherein the shape memory wire (2) is configured to convert the electrical energy of the current flowing through it into thermal energy and to change its length from a first length to a second length under the influence of this heat,
with a wire insulation (3) which surrounds at least some sections of the shape memory wire (2) and consists of an electrically insulating material, with a flexible sheath (3) which is pressure-resistant in the longitudinal direction of the shape memory wire (2), which forms a guide for the shape memory wire (2) and a counterbearing for supporting the tensile forces to be transmitted which are effective as a result of the change in shape of the shape memory wire (2), and which surrounds the wire insulation (3), **characterized in that** the at least two actuators (1, 1a, 1b, 1c, 1d, 1e, 1f) are arranged in the shaft (11, 21) so that the shape memory wires (2) of the actuators (1, 1a, 1b, 1c, 1d, 1e, 1f) are offset from another in the axial direction of the shaft and are free of any overlapping in the axial direction.

2. Endoscopic probe according to claim 1, **characterized in that** the change in length of the shape memory wire (2) under the influence of the heat is generated by the current flowing through it is a contraction.

3. Endoscopic probe according to claim 1 or 2, **characterized in that** the wire insulation (3) forms a flexible guide tube between the shape memory wire (2) and the sheath (4).

4. Endoscopic probe according to claim 1, 2 or 3, **characterized in that** the wire insulation (3) is made of polyimide.

5. Endoscopic probe according to one of the preceding claims, **characterized in that** the sheath (4) is formed by a strip or a wire that is wound into a cylindrical spiral.

6. Endoscopic probe according to claim 5, **characterized in that** the spiral is wound out of metal.

7. Endoscopic probe according to one of the preceding claims, **characterized in that** the sheath (4) is provided with a sheath insulation surrounding the sheath (4), which thermally insulates the sheath (4) from its surroundings.

8. Endoscopic probe according to one of the preceding claims, **characterized in that** one end of the shape memory wire (2) is mechanically fixed at a counterholder (7, 7a) and that the sheath (4) is also secured with its end at the counterholder (7, 7b).

9. Endoscopic probe according to claim 8, **characterized in that** the shape memory wire (2) is connected to the the counterholder (7, 7a) with the electrical conductor (5).

10. Endoscopic probe according to claim 8 or 9, **characterized in that** a mechanical pull cable (23) is connected at the end of the shape memory wire (2) facing away from the counterholder (7, 7a).

11. Endoscopic probe according to claim 10, **characterized in that** the shape memory wire (2) is connected to the pull cable (23) via a sleeve (24).

12. Endoscopic probe according to claim 10 or 11, **characterized in that** at least some sections of the pull cable (23) are also surrounded by the wire insulation (3) and the sheath (4).

13. Endoscopic probe according to one of the preceding claims, **characterized in that** the shape memory wire (2) is connected at one end to the electrical conductor (5) and at the other end to a reference potential.

14. Endoscopic probe according to claim 1, **characterized in that** the actuator (1, 1a, 1b, 1c, 1d, 1e, 1f) is aligned with its longitudinal axis essentially parallel to the longitudinal axis of the shaft (11, 21).

15. Endoscopic probe according to one of claims 1 to 14, **characterized in that** it is provided with a power supply (14, 27) and that it is provided with a control device (15, 28) which supplies the actuators (1, 1a, 1b, 1c, 1d, 1e, 1f) for moving the shaft head (12, 22) with current in such a manner that the actuators (1, 1a, 1b, 1c, 1d, 1e, 1f) deflect the shaft head (12, 22) out of a specified starting position and move it into a specified end position, and move the shaft head (12, 22) out of the specified end position back into its starting position.

16. Endoscopic probe according to claim 15, **characterized in that** the control device (15, 28) is configured to determine the resistance of the shape memory wire (2) of the actuator (1, 1a, 1b, 1c, 1d, 1e, 1f) or of the actuators (1, 1a, 1b, 1c, 1d, 1e, 1f) and to determine from the resistance the actual state of the deflection of the shape memory wire (2) to compare this with a specified target state of the deflection of the shape memory wire (2).

17. Endoscopic probe according to any one of claims 1 to 16, **characterized in that** it is provided with at least one temperature sensor (17, 18).

18. Endoscopic probe according to claim 17, **characterized in that** it is provided with at least a first temperature sensor (17) that determines the temperature at the shape memory wire (2) of the actuator or actuators (1, 1a, 1b, 1c, 1d, 1e, 1f).

19. Endoscopic probe according to claim 17 or 18, **characterized in that** it is provided with a second temperature sensor (18) that determines the ambient temperature of the shaft (11, 21) or of the shaft head (12, 22).

20. Endoscopic probe according to any one of claims 15 to 19, **characterized in that** it is provided with an even number of actuators (1, 1a, 1b, 1c, 1d, 1e, 1f), of which two actuators (1, 1a, 1b, 1c, 1d, 1e, 1e, 1f) in each case form an actuator pair with a first actuator and a second actuator, wherein the two actuators (1, 1a, 1b, 1c, 1d, 1e, 1f) of an actuator pair both expand their shape memory wires (2) to a specified second length.

21. Endoscopic probe according to any one of claims 1 to 20, **characterized in that** the shaft (11) has an oblong cross-section.

22. Endoscopic probe according to claims 1 to 21, **characterized in that** the shaft head (12) can be turned relative to the shaft (11) around a shaft head rotation axis that is aligned essentially perpendicular to the longitudinal axis of the shaft (11).

23. Endoscopic probe according to claim 22, **characterized in that** the shaft head (12) has the form a a rotary plate.

24. Endoscopic probe according to one of claims 1 to 23, **characterized in that** the shape memory wire (2) of the actuator (1, 1a, 1b, 1c, 1d, 1e, 1f) is directly attached to the shaft head (12).

25. Endoscopic probe according to one of claims 1 to 21, **characterized in that** the shaft head (22) has several ring or tube-shaped shaft head segments (25) that can be moved relative to one another, which can be aligned by the actuator or actuators (1c, 1d, 1e, 1f).

26. Endoscopic probe according to claim 25, **characterized in that** the shaft head segments (25) are made of plastic.

27. Endoscopic probe according to claim 25 or 26, **characterized in that** the shaft head segments (25) have openings through which the shape memory wires (2) or pull cables (23) connected to the shape memory wires (2) of the actuators (1c, 1d, 1e, 1f) are guided.

## Revendications

1. Sonde endoscopique
avec un tube longitudinal (11, 21) qui es conçue comme un corps creux,
avec une extrémité proximale du tube (11, 21),
avec une extrémité distale du tube (11, 21),
avec une tête du tube (12, 22) à l'extrémité distale du tube (11, 21), la tête (12, 22) étant mobile par rapport au tube (11, 21),
avec au moins deux actionneurs, conçus pour orienter la tête (12, 22) par rapport au tube (11, 21),
chaque actionneur possédant
un fil à mémoire de forme (2) en alliage à mémoire de forme,
avec un conducteur électrique (5), relié de façon électriquement conductrice au fil à mémoire de forme (2) et conçu pour alimenter le fil à mémoire de forme (2) en courant,
le fil à mémoire de forme (2) étant conçu pour convertir l'énergie électrique du courant qui le traverse en énergie thermique et pour changer, sous l'action de cette chaleur, sa première longueur en une deuxième longueur,
une isolation du fil (3) en matériau électriquement isolant qui entoure au moins certains segments du fil à mémoire de forme (2),
une gaine (3) flexible résistant à la pression dans l'axe longitudinal du fil à mémoire de forme (2), qui constitue un guidage pour le fil (2) et un appui pour les forces de traction à transmettre agissant sous l'effet du changement de forme du fil (2) et qui entoure l'isolation du fil (3) **caractérisée en ce que**,
les au moins deux actionneurs (1, 1a, 1b, 1c, 1d, 1e, 1f) sont disposés dans le tube (11, 21) de telle façon que les fils à mémoire de forme (2) des actionneurs (1, 1a, 1b, 1c, 1d, 1e, 1f) sont décalés entre eux dans la direction axiale du tube et ne présentent pas de chevauchement dans la direction axiale.

2. Sonde endoscopique selon la revendication 1, **caractérisée en ce que** le changement de longueur du fil à mémoire de forme (2) sous l'action de la chaleur produite par le courant électrique qui le traverse est une contraction.

3. Sonde endoscopique selon la revendication 1 ou 2, **caractérisée en ce que** l'isolation du fil (3) constitue un tube coulissant flexible entre le fil à mémoire de forme (2) et la gaine (4).

4. Sonde endoscopique selon la revendication 1, 2 ou 3, **caractérisée en ce que** l'isolation du fil (3) est en polyimide.

5. Sonde endoscopique selon l'une des revendications précédentes, **caractérisée en ce que** la gaine (4) est formée d'une bande ou d'un fil, enroulé en une spirale cylindrique.

6. Sonde endoscopique selon la revendication 5, **caractérisée en ce que** la spirale est enroulée à partir de métal.

7. Sonde endoscopique selon l'une des revendications précédentes, **caractérisée en ce que** la gaine (4) est dotée d'une isolation qui entoure cette gaine (4) et l'isole thermiquement contre l'environnement.

8. Sonde endoscopique selon l'une des revendications précédentes, **caractérisée en ce qu'**une extrémité du fil à mémoire de forme (2) est mécaniquement attachée à un support (7, 7a) et **en ce que** la gaine (4) est également fixée au support (7, 7b) sur une extrémité.

9. Sonde endoscopique selon la revendication 8, **caractérisée en ce que** le fil à mémoire de forme (2) est relié au support (7, 7a) par le conducteur électrique (5).

10. Sonde endoscopique selon la revendication 8 ou 9, **caractérisée en ce qu'**un câble de traction mécanique (23) est relié à l'extrémité du fil à mémoire de forme (2) qui se trouve à l'opposé du support (7, 7a).

11. Sonde endoscopique selon la revendication 10, **caractérisée en ce que** le fil à mémoire de forme (2) est relié au câble de traction (23) par une gaine (24).

12. Sonde endoscopique selon la revendication 10 ou 11, **caractérisée en ce que** le câble de traction (23) est également entouré, au moins sur certains segments, de l'isolation du fil (3) et de la gaine (4).

13. Sonde endoscopique selon l'une des revendications précédentes, **caractérisée en ce que** le fil à mémoire de forme (2) est relié à une extrémité au conducteur électrique (5) et à l'autre extrémité à un potentiel de référence.

14. Sonde endoscopique selon la revendication 1, **caractérisée en ce que** l'actionneur (1, 1a, 1b, 1c, 1d, 1e, 1f) avec son axe longitudinal est essentiellement parallèle à l'axe longitudinal du tube (11, 21).

15. Sonde endoscopique selon l'une des revendications 1 à 14, **caractérisée en ce qu'**elle est dotée d'une alimentation en tension (14, 27) et **en ce qu'**elle est munie d'un dispositif de commande (15, 28), qui alimente en courant les actionneurs (1, 1a, 1b, 1c, 1d, 1e, 1f) faisant bouger la tête de tube (12, 22) de telle façon que ces actionneurs (1, 1a, 1b, 1c, 1d, 1e, 1f) déplacent la tête (12, 22) à partir d'une position initiale définie pour la mettre dans une position finale définie, puis déplacent la tête (12, 22) de nouveau en sens inverse.

16. Sonde endoscopique selon la revendication 15, **caractérisée en ce que** le dispositif de commande (15, 28) est conçu pour déterminer la résistance du fil à mémoire de forme (2) de l'actionneur (1, 1a, 1b, 1c, 1d, 1e, 1f) ou des actionneurs (1, 1a, 1b, 1c, 1d, 1e, 1f) et, à partir de la résistance, l'état réel de l'excursion du fil à mémoire de forme (2) afin de le comparer à un état théorique prédéfini de l'excursion du fil à mémoire de forme (2).

17. Sonde endoscopique selon l'une des revendications 1 à 16, **caractérisée en ce qu'**elle est munie au moins d'un capteur de température (17, 18).

18. Sonde endoscopique selon la revendication 17, **caractérisée en ce qu'**elle est dotée au moins d'un premier capteur de température (17), qui détermine la température au niveau du fil à mémoire de forme (2) de l'actionneur ou des actionneurs (1, 1a, 1b, 1c, 1d, 1e, 1f).

19. Sonde endoscopique selon la revendication 17 ou 18, **caractérisée en ce qu'**elle est équipée d'un deuxième capteur de température (18), qui détermine la température ambiante du tube (11, 21) ou de sa tête (12, 22).

20. Sonde endoscopique selon l'une des revendications 15 à 19, **caractérisée en ce qu'**elle est munie d'un nombre pair d'actionneurs (1, 1a, 1b, 1c, 1d, 1e, 1f), parmi lesquels respectivement deux actionneurs (1, 1a, 1b, 1c, 1d, 1e, 1f) forment une paire, avec un premier actionneur et un deuxième actionneur, les deux actionneurs (1, 1a, 1b, 1c, 1d, 1e, 1f) d'une paire dilatant leurs fils à mémoire de longueur (2) mutuellement à une deuxième longueur prédéfinie.

21. Sonde endoscopique selon l'une des revendications 1 à 20, **caractérisée en ce que** le tube (11) présente une section transversale longitudinale.

22. Sonde endoscopique selon la revendication 1 à 21, **caractérisée en ce que** la tête de tube (12) peut tourner par rapport au tube (11) autour d'un axe essentiellement perpendiculaire à l'axe longitudinal du tube (11).

23. Sonde endoscopique selon la revendication 22, **caractérisée en ce que** la tête de tube (12) a la forme d'un plateau tournant.

24. Sonde endoscopique selon l'une des revendications 1 à 23, **caractérisée en ce que** le fil à mémoire de forme (2) de l'actionneur (1, 1a, 1b, 1c, 1d, 1e, 1f) est directement fixé à la tête de tube (12).

25. Sonde endoscopique selon l'une des revendications 1 à 21, **caractérisée en ce que** la tête de tube (22) présente plusieurs segments (25) de forme annulaire ou tubulaire mobiles l'un par rapport à l'autre, qui sont orientés par l'actionneur ou les actionneurs (1c, 1d, 1e, 1f).

26. Sonde endoscopique selon la revendication 25, **caractérisée en ce que** les segments de tête de tube (25) sont en plastique.

27. Sonde endoscopique selon la revendication 25 ou 26, **caractérisée en ce que** les segments de tête de tube (25) présentent des ouvertures de passage à travers lesquelles sont guidés les fils à mémoire de forme (2) ou les câbles de traction (23) couplés aux fils à mémoire de forme (2) des actionneurs (1c, 1d, 1e, 1f).
